# EUROPÄISCHE PATENTSCHRIFT

(11) **EP 1 182 196 B1**
(45) Veröffentlichungstag und Bekanntmachung des Hinweises auf die Patenterteilung: **30.06.2004**
(21) Anmeldenummer: 00118441.5
(22) Anmeldetag: 24.08.2000
(51) Int. Cl.: C07D 233/54, C07D 213/20

(54) **Halogenidfreie Herstellung ionischer Flüssigkeiten**
Halogen-free preparation of ionic fluids
Préparation sans halogénures de fluides ioniques

(43) Veröffentlichungstag der Anmeldung: 27.02.2002
(73) Patentinhaber: Solvent Innovation GmbH, 50679 Köln (DE)
(72) Erfinder: Wasserscheid, Peter, 50829 Köln (DE); Hilgers, Claus, 50679 Köln (DE); Boesmann, Andreas, D-52068 Aachen (DE)
(74) Vertreter: Weber, Thomas, Dr. Dipl.-Chem.

(56) Entgegenhaltungen:
- WO-A-97/02252
- GB-A- 2 337 754

## Beschreibung

Die Erfindung betrifft ein Verfahren zur Herstellung ionischer Flüssigkeiten der allgemeinen Formel

[A]ₙ⁺[Y]ⁿ⁻,

(n=1 oder 2)
durch Alkylierung der zugrundeliegenden Amine, Phosphine, Imidazole, Pyrazole, Triazole oder Pyridine mit einem Disulfat der allgemeinen Formel R⁴-SO₄-R⁵ und nachfolgendem und Austausch des Sulfatanions.

Unter ionischen Flüssigkeiten versteht man Salze oder Gemische aus Salzen, deren Schmelzpunkte unterhalb 80°C liegen. Diese Salze bestehen aus Anionen wie z.B. Halogenostannaten, Halogenoaluminaten, Hexafluorophosphaten oder Tetrafluoroboraten kombiniert mit substituierten Ammonium-, Phosphonium, Pyridinium-, Triazolium-, Pyrazolium- oder Imidazolium-Kationen. Mehrere Veröffentlichungen beschreiben bereits die Verwendung ionischer Flüssigkeiten als Lösungsmittel für Übergangsmetall-katalysierte Reaktionen (Übersichtsartikel: T. Welton, *Chem. Rev.* **1999,** *99*, 2071). Beispielsweise wurden Hydrierungen von Olefinen mit Rhodium(I) (P. A. Z. Suarez, J. E. L. Dullius, S. Einloft, R. F. de Souza und J. Dupont, *Polyhedron 15*/*7*, **1996,** 1217-1219), Ruthenium(II) und Cobalt(II) komplexen (P. A. Z. Suarez, J. E. L. Dullius, S. Einloft, R. F. de Souza und J. Dupont, *Inorganica Chimica Acta 255,* **1997,** 207-209) in ionischen Flüssigkeiten mit Tetrafluoroborat-Anion erfolgreich bearbeitet. Auch die Hydroformylierung von funktionalisierten und unfunktionalisierten Olefinen gelingt mit Rhodium-Katalysatoren in ionischen Flüssigkeiten mit schwach koordinierenden Anionen (z.B. PF₆⁻, BF₄⁻), EP-A-0776880, Y. Chauvin, L. Mussmann, H. Olivier, (*Angew. Chem., Int. Ed. Engl.,* **1995,** *34*, 2698; W. Keim, D. Vogt, H. Waffenschmidt, P. Wasserscheid, *J. of Cat.,* **1999,** *186,* 481).

Ein weiteres wichtiges Einsatzfeld ionischer Flüssigkeiten liegt in ihrer Verwendung als Extraktionsmittel zur Stofftrennung (J.G. Huddleston, H.D. Willauer, R.P.Swatloski, A.E. Visser, R.D. Rogers, *Chem. Commun.* **1998,** 1765-1766; b) A.E. Visser, R.P. Swatlowski, R.D. Rogers, *Green Chemistry* **2000,** *2(1),* 1-4). Für diesen Anwendungsbereich sind solche ionischen Flüssigkeiten von besonderem Interesse, die mit Wasser nur begrenzt mischbar sind.

Allgemein gilt, dass ionische Flüssigkeiten - vor allem für die Verwendung als Lösungsmittel in der Übergangsmetallkatalyse - spezielle Reinheitsanforderungen erfüllen müssen. Viele Übergangsmetallkatalysatoren sind z. B. empfindlich gegenüber Feuchtigkeitsspuren und Spuren von Halogenidionen. Da sie in nur sehr geringen Mengen der Reaktionslösung zugesetzt werden, muss das Lösungsmittel frei von sämtlichen Spuren dieser Katalysatorgifte sein. Da ionische Flüssigkeiten auf Grund ihrer nicht-flüchtigen Natur nicht wie organische Lösungsmittel vor Gebrauch destillativ gereinigt werden können, muss bereits bei ihrer Synthese eine möglichst hohe Reinheit angestrebt werden.

Die Synthese von Chlorid-, Bromid- und Iodid-freien ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen, ist aus oben genannten Gründen von besonderer technischer Bedeutung, da diese ionischen Flüssigkeiten in der Regel sehr geeignete Lösungsmittel für katalytische Reaktionen mit Übergangsmetallkomplexen darstellen und außerdem eine geringe Löslichkeit in Wasser aufweisen. Vor dem Hintergrund der speziellen technischen Bedeutung dieser ionischen Flüssigkeiten müssen geeignete Verfahren zu ihrer Synthese problemlos in einen größeren Produktionsmaßstab (> 5Liter) übertragen werden können.

Zur Synthese von ionische Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen wird bei den bisher beschriebenen Verfahren zunächst durch Reaktion eines Amins NR₁R₂R₃, eines Phosphans PR¹R²R³, eines Imidazolderivates der allgemeinen Formel R¹R²⁺N=CR³-R⁵-R³C=N⁺R¹R² oder eines Pyridiniumderivates der allgemeinen Formel R¹R²N=CR³R⁴⁺ mit einem Alkylchlorid, Alkylbromid oder Alkyliodid das entsprechende Halogenidsalz [Kation]⁺X⁻ gebildet und isoliert (F.H. Hurley, T.P. Wier, Jr., *J. Electrochem. Soc.* **1951,** *98*, 207-212; J.S. Wilkes, J.A. Levisky, R.A. Wilson, C.L. Hussey, *Inorg. Chem.* **1982,** *21,* 1263-1264; A.A.K. Abdul-Sada, P.W. Ambler, P.K.G. Hodgson, K.R. Seddon, N.J. Steward, *WO 95*/*21871,* **1995;** b) R.H. Dubois, M.J. Zaworotko, P.S. White, *Inorg. Chem.* **1989,** *28,* 2019-2020; J.F. Knifton, *J. Mol. Catal.* **1987,** *43,* 65-78; C.P.M. Lacroix, F.H.M. Dekker, A.G. Talma, J.W.F. Seetz, *EP 989134.* **1998**).

Ausgehend vom gebildeten und isolierten Halogenidsalz [A]⁺X⁻ sind zwei unterschiedliche Wege zur Synthese von ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen bekannt. Zum einen wird das Halogenidsalz durch Zugabe eines Metallsalzes MY (unter Ausfällung oder Abscheidung des Salzes MX oder des Produktes [A]⁺[Y]⁻ aus dem jeweils verwendeten Lösungsmittel) umgesetzt - wobei [Y]⁻ ein Hexafluorophosphat-, Tetrafluoroborat-, Bis(trifluormethylsulfonyl)amid-, Perfluoralkylsulfonat- und Perfluoralkylcarboxylat-Ion darstellt und M⁺ für ein Alkalikation steht (J.S. Wilkes, M.J. Zaworotko, *J. Chem. Soc. Chem. Commun.* **1992,** 965-967; Y. Chauvin, L. Mußmann, H. Olivier, *Angew. Chem.* **1995,** 107, 2941-2943; P.A.Z. Suarez, J.E.L. Dullius, S. Einloft, R.F. de Souza, J. Dupont, *Polyhedron,* **1996,** *15*, 1217-1219; P. Bonhöte, A.-P. Dias, N. Papageorgiou, K. Kalyanasundaram, M. Grätzel, *Inorg. Chem.* **1996,** *35*, 1168-1178; C.M. Gordon, J.D. Holbrey, A.R. Kennedy, K.R. Seddon, *J. Mater. Chem.* **1998,** *8*, 2627-2638; P.A.Z. Suarez, S. Einloft, J.E.L. Dullius, R.F. de Souza, J. Dupont, *J. Chim. Phys.* **1998,** *95*, 1626-1639; A.J. Carmichael, C. Hardacre, J.D. Holbrey, M. Nieuwenhuyzen, K.R. Seddon, *Anal. Chem.* **1999,** *71,* 4572-4574; J.D. Holbrey, K.R. Seddon, *J. Chem. Soc., Dalton Trans.* **1999,** 2133-2140). Zum anderen wird durch Zugabe einer starken Säure H⁺ [Y]⁻ das Halogenidion unter Freisetzung von H⁺ X⁻ verdrängt und gegen [Y]⁻ ausgetauscht - wobei [Y]⁻ hier für ein Hexafluorophosphat-, Tetrafluoroborat-, Bis(trifluormethylsulfonyl)amid-, Perfluoralkylsulfonat- und Perfluoralkylcarboxylat-Ion steht (J. Fuller, R.T. Carlin, H.C. de Long, D. Haworth, *J. Chem. Soc. Chem. Commun.* **1994,** 299-300).

Vor dem Hintergrund einer angestrebten Synthese von Chlorid-, Bromid- und Iodid-freien ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen,

Vor allem in Hinblick auf die Durchführung im größeren Maßstab (> 5 Liter) besitzen die oben erwähnten Verfahren des Standes der Technik zahlreiche gravierende Nachteile.

Die zunächst durchgeführte Quarternisierung eines Amins, Imidazolderivats, Pyridins oder Phosphans mit einem Alkylchlorid zur Bildung des entsprechende Chloridsalzes [A]⁺Cl⁻ ist eine sehr langsame Reaktion, die erhöhte Reaktionstemperaturen erfordert. In der Regel muss bei mindestens 50°C gearbeitet werden, um ausreichende Reaktionsgeschwindigkeiten zu erzielen. Je nach verwendetem Alkylchlorid erfordert dies eine Arbeitsweise im Druckreaktor. Dennoch liegen die Reaktionszeiten für beispielsweise die Umsetzung von 4-Picolin mit Butylchlorid bei 130h (70°C), um zu einem Umsatz von 50% zu gelangen (P. Wasserscheid, *Dissertation,* **1998**, RWTH Aachen). Für die technische Produktion der Chloridsalze stellen sowohl die sehr langen Reaktionszeiten, als auch die eventuell notwendige Druckreaktion erhebliche Kostenfaktoren dar.

Bei der Verwendung von Alkylbromiden und Alkyliodiden als Alkylierungsmittel zur Darstellung der entsprechenden Bromid- und Iodidsalze [A]⁺Br⁻ bzw. [A]⁺I⁻ sind Reaktionszeiten und -temperaturen zwar deutlich geringer, allerdings sind die Alklylbromide bzw. Alkyliodide, die hierbei verwendet werden müssen, in den meisten Fällen deutlich teurer als die entsprechenden Alkylchloride.

Zudem ist die Löslichkeit von Erd-/Alkaliiodiden und bromiden besser in dem Reaktionslösungsmittel, was eine vollständige Abtrennung signifikant erschwert.

In vielen Fällen bereitet die Isolierung der gebildeten Halogenidsalze [A]⁺X⁻ erhebliche Probleme. Nicht umgesetzte, schwerflüchtige Alkylhalogenide sind nur mit erheblichem Aufwand (Zeit, Energie) destillativ vollständig abzutrennen. Außerdem sind die Halogenidsalze selbst in fast allen Fällen Feststoffe, die durch Filtration isoliert werden müssen. Gerade bei Synthesen im größeren Maßstab (< 5 kg) sind damit erhebliche Probleme verbunden (z.B. Verstopfung der Filter).

Bei der weiteren Umsetzung des isolierten Halogenidsalzes [A]⁺X⁻ zu ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen ergeben sich beim Arbeiten nach den bekannten Verfahren weitere Probleme, die insbesondere eine Produktion der Chlorid-, Bromid- und Iodidfreien ionischen Flüssigkeiten in größerem Maßstab (>5 Liter) sehr kostenintensiv machen.

Wird das Halogenidsalz durch Zugabe eines Metallsalzes M⁺[Y]⁻ oder einer starken Säure H⁺ [Y]⁻ gegen das Anion [Y]⁻ ausgetauscht, so ist die Löslichkeit des verwendeten Halogenid-Ions in der ionischen Flüssigkeit bzw. in der, die ionischen Flüssigkeit enthaltenden Phase, entscheidendes Kriterium für die Reinheit der erhaltenen ionischen Flüssigkeit. Erhebliche Probleme bereitet die Tatsache, dass die in der Regel schlecht wasserlöslichen ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen häufig eine erhebliche Löslichkeit für Halogenidionen aufweisen. Dadurch ergibt sich eine Halogenidionen-Verunreinigung des Produkts, die entweder eine sehr aufwendige Nachreinigung (etwa mit Silbersalzen) oder eine sehr eingeschränkte Verwendbarkeit und damit einen entsprechend niedrigen Wert des Produkts zur Folge hat.

Aufgabe der vorliegenden Erfindung war es dementsprechend ein Verfahren zur Herstellung ionischer Flüssigkeiten zu Verfügung zu stellen, welches die oben genannten Nachteile nicht aufweist und zu Produkten führt, welche in hoher Reinheit und Ausbeute als unmittelbares Verfahrensprodukt zur Verfügung stehen, und zudem ein leichtes Scale-Up ermöglicht.
1. Die Aufgabe wurde erfindungsgemäß gelöst durch ein Verfahren zur Herstellung ionischer Flüssigkeiten der allgemeinen Formel

   [A]ₙ⁺ [Y]ⁿ⁻,

   wobei n = 1 oder 2 ist und
   das Anion [Y]ⁿ⁻ ausgewählt ist aus der Gruppe bestehend aus Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprate (I) und (II), Sulfat ([SO₄]²⁻), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann,
   das Kation [A]⁺ ist ausgewählt aus
   - quarternären Ammonium-Kationen der allgemeinen Formel

      [NR¹R²R³R]⁺,
   - Phosphonium-Kationen der allgemeinen Formel

      [PR¹R²R³R]⁺,
   - Imidazolium-Kationen der allgemeinen Formel 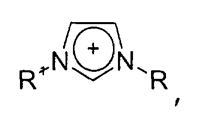 wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
   - Pyridinium-Kationen der allgemeinen Formel 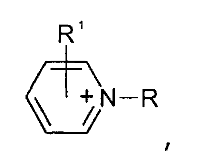 wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
   - Pyrazolium-Kationen der allgemeinen Formel 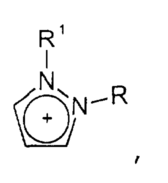 wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
   - und Triazolium-Kationen der allgemeinen Formel 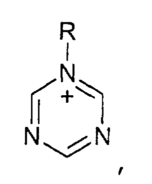 wobei der Triazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
   und die Reste R¹, R², R³ unabhängig voneinander ausgewählt sind aus der Gruppe bestehend aus
   - Wasserstoff;
   - linearen oder verzweigten, gesättigten oder ungesättigten, aliphatischen oder alicyclischen Alkylgruppen mit 1 bis 20 Kohlenstoffatomen;
   - Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
   - Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
dadurch gekennzeichnet, dass die zugrundeliegenden Amine, Phosphine, Imidazole, Pyridine, Triazole und Pyrazole mit einem Disulfat der allgemeinen Formel R⁴-SO₄-R⁵ (Organodisulfat) alkyliert werden, wobei die Reste R⁴ und R⁵ unabhängig voneinander ausgewählt sein können aus
- linearen oder verzweigten, gesättigten oder ungesättigten, aliphatischen oder alicyclischen Alkylgruppen mit 1 bis 24 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 24 Kohlenstoffatomen im Arylrest, die gegebenenfalls mit wenigstens einer C₁-C₆-Alkylgruppe und/oder einem Halogenenatomen substituiert sein können,
alkyliert werden und anschließend ein Austausch des Sulfatanions R⁴-SO₄⁻ oder R⁵-SO₄⁻ durch das oben definierte Anion [Y]⁻ oder [Y]²⁻ erfolgt.

Weitere bevorzugte Ausgestaltungen ergeben sich aus den Patentansprüchen.

Durch die Alkylierung mit Hilfe von Diorganosulfaten der allgemeinen Formel R⁴-SO₄-R⁵ anstelle der bisher ausschließlich als Alkylierungsmittel zur Synthese ionischen Flüssigkeiten verwendeten Alkylchloride, Alklybromide oder Alklyhalogenide können die oben beschriebenen Nachteile der Verfahren des Standes der Technik umgangen werden.

Zur Durchführung des erfindungsgemäßen Verfahrens werden die zugrundeliegenden Amine, Phosphine, Imidazole, Pyrazole, Triazole oder Pyridine in Substanz oder in einem inerten Lösungsmittel gelöst mit dem Diorganosulfat versetzt. Vorzugsweise werden Disulfate der allgemeinen Formel R⁴-SO₄-R⁵ eingesetzt, in welchen die Gruppe R⁴ der Gruppe R⁵ entspricht, also symmetrische Organodisulfate der allgemeinen Formel R⁴-SO₄-R⁴, in welcher R⁴ die obengenannte Bedeutung hat. Bevorzugt sind Di-C₁-C₆-Alkylsulfate, insbesondere Dimethyl-, Diethyl-, Di-n-propyl-, Diisopropyl-, Di-n-butyl-, Diisobutyl-, Di-tert.-butyl-, Di-n-pentyl-, Diisopentyl-, Di-neo-pentyl-, Di-n-hexylsulfat sowie Dicyclohexylsulfat.

In Zusammenhang mit der Erfindung werden die Begriffe Diorganosulfat und Disulfat austauschbar verwendet. Der Begriff Alkylierung bezieht sich auf die Einführung der obengenannten Gruppen R⁴ oder R⁵.

Das molare Verhältnis des Diorganosulfats zu den zugrundeliegenden Aminen, Phosphinen, Imidazolen-, Triazolen-, Pyrazolen-, oder Pyridinen liegt zwischen 1 und 5, bevorzugt zwischen 1 und 1,5, besonders bevorzugt zwischen 1 und 1,1.

Im Vergleich zur Alkylierung mit Alkylhalogeniden weisen die Diorganosulfate eine deutlich höhere Reaktivität auf. Die Reaktionszeit einer typischen Umsetzung beträgt zwischen 1 min und 8 h, bevorzugt 5 min bis 60 min, besonders bevorzugt 15 bis 30 min..

Die Alkylierungsreaktion mit dem Disulfat kann bei Temperaturen zwischen -20 und 250°C, bevorzugt 0 °C bis 180 °C, besonders bevorzugt jedoch bei RT (20°C) durchgeführt werden, was im Vergleich zum Stand der Technik zu einer deutlichen Verminderung des apparativen und Verfahrensaufwandes führt.

Die durch die erfindungsgemäß durchgeführte Alkylierungsreaktion erhaltenen Verbindungen der allgemeinen Formel [A]⁺[R⁵-SO₄]⁻ oder [A]⁺[R⁴-SO₄]⁻ können in einer Ausführungsform durch die gängigen, dem Fachmann bekannten Verfahren isoliert werden, bevor sie in die Anionenaustauschreaktion eingesetzt werden. Dies empfiehlt sich bevorzugt dann, wenn die Alkylierungsreaktion unter Zugabe inerter Lösungsmittel erfolgte.

Eine weitere Ausführungsform des erfindungsgemäßen Verfahrens sieht die sofortige Umsetzung der Organosulfatsalze [A]⁺[R⁵-SO₄]⁻ oder [A]⁺[R⁴-SO₄]⁻ ohne vorherige Isolierung mit der wässrigen Lösung des Metallsalzes M^{m+}[Y]⁻ₘ, M₂⁺[Y]²⁻ oder M²⁺[Y]²⁻ vor.

Unabhängig von der oben beschriebenen Ausführungsweise kann der Anionenaustausch bei einer Temperatur zwischen -10 und 100°C, bevorzugt zwischen 0 und 80°C, besonders bevorzugt jedoch bei RT durchgeführt werden, wodurch auch dieser Reaktionsschritt ein deutlich verringerten apparativen Aufwand erfordert.

In einer besonderen Ausgestaltung der Erfindung können die Alkyl-, Aryl-, Arylalkyl- und Alkylaryl-Sulfonatgruppen (Anion [Y]) durch Halogenatome, insbesondere Fluor, Chlor oder Brom substituiert sein. Besonders bevorzugt sind die fluorierten, insbesondere die perfluorierten Alkyl- und obengenannten Arylsulfonate, wie das Trifluormethansulfonat (Triflat). Als nicht halogenierte Vertreter sind die Methansulfonat-, Benzolsulfonat- und die Toluolsulfonat-Gruppe zu nennen, sowie alle weiteren im Stand der Technik bekannten Sulfonat-Austrittsgruppen.

In einer weiteren Ausgestaltung der Erfindung können die Alkyl-, Aryl-, Arylalkyl- und Alkylaryl-Carboxylatgruppen durch Halogenatome, insbesondere Fluor, Chlor oder Brom substituiert sein. Besonders bevorzugt sind die fluorierten, insbesondere die perfluorierten Alkyl - und obengenannten Arylcarboxylate, wie das Trifluormethancarboxylat (Trifluoracetat; CF₃COO⁻). Als nicht halogenierte Vertreter sind die Acetat- und Benzoat-Gruppe zu nennen, sowie alle weiteren im Stand der Technik bekannten Carboxylat-Austrittsgruppen.

In bevorzugten Ausgestaltungen der Erfindung können die im Zusammenhang mit den Substituenten erwähnten C₁-C₆-Alkyl-Gruppen jeweils unabhängig voneinander durch C₂-C₄-Alkyl-Gruppen ersetzt werden. Ebenso können die im Zusammenhang mit den Substituenten erwähnten C₁-C₆-Alkoxy-Gruppen jeweils unabhängig voneinander durch C₂-C₄-Alkoxy-Gruppen ersetzt werden. In einer weiteren Alternative der Erfindung können die im Zusammenhang mit den Substituenten erwähnten C₅-C₁₂-Aryl-Gruppen jeweils unabhängig voneinander durch C₆-C₁₀-Aryl-Gruppen, die C₃-C₈-Heteroaryl-Gruppen jeweils unabhängig voneinander durch C₃-C₆-Heteroaryl-Gruppen ersetzt werden. Die Halogenatome, mit welchen die Alkyl-, Alkoxy- und Aryl-Gruppen substituiert sein können sind ausgewählt aus Fluor, Chlor, Brom und Iod, vorzugsweise Fluor, Chlor und Brom.

Ein einer bevorzugten Ausgestaltung ist der Rest R' ein linearer oder verzweigter 1 bis 8 Kohlenstoffatome enthaltender aliphatischer oder alicyclischer Alkyl- oder ein C₆-C₁₀-Aryl-, C₆-C₁₀-Aryl-C₁-C₄-alkyl- oder C₁-C₄-Alkyl-C₆-C₁₀-Arylrest, der durch Halogenatome substituiert sein kann.

Die Kationen sind vorzugsweise ausgewählt aus Trimethylphenylammonium, Methyltrioctylammonium, Tetrabutylphosphonium, 3-Butyl-1-methyl-imidazolium, 3-Ethyl-1-methylimidazolium, N-Butylpyridinium, N-Ethylpyridinium, Diethylpyrazolium, 1-Ethyl-3-methylimidazolium, 1-Butyl-3-methylimidazolium, 1-Hexyl-3-methylimidazolium, 1-Octyl-3-methylimidazolium, 1-,Decyl-3-methylimidazolium, 1-Butyl-4-methylpyridinium, 1-Butyl-3-methylpyridinium, 1-Butyl-2-methylpyridinium, 1-Butyl-pyridinium.

Gemäß dem erfindungsgemäßen Verfahren lässt man das Disulfat der Formel R⁴-SO₄-R⁵, insbesondere das symmetrische Disulfat bevorzugt reagieren mit
- Aminen der allgemeinen Formel

   NR¹R²R³,
- Phosphanen der allgemeinen Formel

   PR¹R²R³,
- Imidazolen der allgemeinen Formel 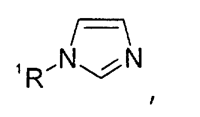 wobei der Imidazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinen der allgemeinen Formel 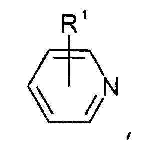 wobei der Pyridin-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolen der allgemeinen Formel 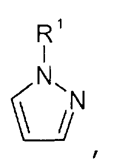 wobei der Pyrazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- oder Triazolen der allgemeinen Formel 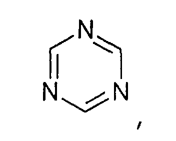 wobei der Triazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen.

Anschließend erfolgt der Austausch des Sulfatanions R⁴-SO₄⁻ oder R⁵-SO₄⁻ durch Zugabe eines Metallsalzes der allgemeinen Formel M^{m+}[Y]⁻ₘ, M₂⁺[Y]²⁻ oder M²⁺[Y]²⁻ unter Bildung der gewünschten binären ionischen Flüssigkeit [A]ₙ⁺ [Y]ⁿ⁻. Bei dem Metallsalz der Formel M^{m+}[Y]⁻ₘ mit m = 1, 2 oder 3 handelt es sich bevorzugt um Alkali- oder Erdalkalimetall-, Blei- oder Silbersalze. Bei den Metallsalzen M₂⁺[Y]²⁻ oder M²⁺[Y]²⁻ handelt es sich vorzugsweise um Sulfate oder Carbonate der Alkalimetalle oder Kupfer, Nickel, Cobalt, Zink, Magnesium und Eisen. Das Anion Y kann die obengenannten Bedeutungen annehmen.

In einer bevorzugten Ausgestaltung der Erfindung kann das Metallsalz der allgemeinen Formel M^{m+}[Y]⁻ₘ , M₂⁺[Y]²⁻ oder M²⁺[Y]²⁻ als wässrige Lösung eingesetzt werden. Diese wässrigen Lösungen enthalten die Metallsalze in einer Konzentration von 0,1 bis 99 Gew.-%, bevorzugt 20 bis 85 Gew.-%, besonders bevorzugt von 30 bis 75 Gew.-%, bezogen auf die wässrige Lösung.

Das zum Anionenaustausch verwendete Metallsalz der Formel M^{m+}[Y)⁻_{m,} M₂⁺[Y]²⁻ oder M²⁺[Y]²⁻ oder dessen wässrige Lösung wird nach der Alkylierung der Reaktionsmischung zugegeben.

Die Reaktionszeit des Anionenaustauschs beträgt zwischen 1 und 120 Minuten, bevorzugt zwischen 2 und 60 Minuten, besonders bevorzugt zwischen 5 und 15 Minuten. Die Reaktionstemperatur beträgt zwischen -10 und 100°C, vorzugsweise zwischen 0 und 80°C.

Die Alkylierung der Amine, Phosphane, der Imidazole, Pyridinine mit den genannten Diorganosulfaten erfolgt in der Praxis vorteilhafterweise bei Raumtemperatur nach kurzer Reaktionszeit (in der Regel <15min) vollständig. Im Gegensatz zu Alkylbromiden und Alkyliodiden sind die technisch verfügbaren Dialkylsulfate aber deutlich preiswerter. Die meisten der gebildeten Alkylierungsprodukte sind bei Raumtemperatur flüssig und können daher leicht umgefüllt oder gepumpt werden. Da die Reaktion in der Regel quantitativ verläuft, muss kein überschüssiges Diorganosulfat zugesetzt werden. Ein eventuell vorhandener Überschuss an Diorganosulfat wird im zweiten wässrigen Reaktionsschritt problemlos (durch Hydrolyse) abgetrennt, so dass es in keinem Fall zu einer Produktverunreinigung durch das Alkylierungsmittel kommt.

Über die Vorteile bei der Alkylierungsreaktion hinaus bietet die Verwendung von Dialkylsulfaten als Alkylierungsmittel im Rahmen der Synthese von Chlorid-, Bromid- und Iodid-freien ionischen Flüssigkeiten gemäß dieser Erfindung weitere erhebliche Vorteile beim anschließenden Ionenaustausch zur Produktbildung.

Beim Ionenaustausch gemäß dieser Erfindung nutzt man die außerordentlich geringe Löslichkeit von Wasser in ionischen Flüssigkeiten mit Hexafluorophosphat-, Tetrafluoroborat-, Bis(trifluormethylsulfonyl)amid-, Perfluoralkylsulfonat- und Perfluoralkylcarboxylat-Ionen, die außerordentlich gute Wasserlöslichkeit der bei der Alkylierung gebildeten Alkylsulfationen sowie die außerordentlich schlechte Löslichkeit dieser Alkylsulfationen in der gebildeten ionischen Flüssigkeit. Dadurch wird im Verfahren gemäß dieser Erfindung ein außerordentlich schneller und sauberer Ionenaustausch möglich.

Das Alkylsulfatsalz aus der Alkylierungsreaktion wird in Wasser aufgelöst und mit dem obengenannten Metallsalz oder dessen wässriger Lösung versetzt. In der Regel bildet sich sofort eine zweite flüssige Phase der gebildeten ionischen Flüssigkeit. Nach Phasentrennung und Trocknung erhält man die Chlorid-, Bromid- und Iodid-freien ionische Flüssigkeit ohne weitere Reinigungsschritte. Gegebenfalls kann die Wasserphase mit CH₂Cl₂, Chloroform, Chlorbenzol oder Diethylether extrahiert werden, um die Ausbeute an ionischer Flüssigkeit zu verbessern. In allen Reaktionsschritten werden die Reaktanten durch geeignete Maßnahmen gut durchmischt.

Folgende ionische Flüssigkeiten eignen sich im besonderen für eine Darstellung gemäß dem hier beschriebenen Verfahren:
1-Ethyl-3-methylimidazolium tetrafluoroborat
1-Ethyl-3-methylimidazolium hexafluorophosphat
1-Ethyl-3-methylimidazolium-bis-trifluormethansulfonimid
1-Butyl-3-methylimidazolium tetrafluoroborat
1-Butyl-3-methylimidazolium hexafluorophosphat
1-Butyl-3-methylimidazolium-bis-trifluormethansulfonimid
1-Hexyl-3-methylimidazolium tetrafluoroborat
1-Hexyl-3-methylimidazolium hexafluorophosphat
1-Hexyl-3-methylimidazolium-bis-trifluormethansulfonimid
1-Octyl-3-methylimidazolium tetrafluoroborat
1-Octyl-3-methylimidazolium hexafluorophosphat
1-Octyl-3-methylimidazolium-bis-trifluormethansulfonimid
1-Decyl-3-methylimidazolium tetrafluoroborat
1-Decyl-3-methylimidazolium hexafluorophosphat
1-Decyl-3-methylimidazolium-bis-trifluormethansulfonimid
1-Butyl-4-methylpyridinium tetrafluoroborat
1-Butyl-4-methylpyridinium hexafluorophosphat
1-Butyl-4-methylpyridinium-bis-trifluormethansulfonimid
1-Butyl-3-methylpyridinium tetrafluoroborat
1-Butyl-3-methylpyridinium hexafluorophosphat
1-Butyl-3-methylpyridinium-bis-trifluormethansulfonimid
1-Butyl-2-methylpyridinium tetrafluoroborat
1-Butyl-2-methylpyridinium hexafluorophosphat
1-Butyl-2-methylpyridinium-bis-trifluormethansulfonimid
1-Butyl-pyridinium tetrafluoroborat
1-Butyl-pyridinium hexafluorophosphat
1-Butyl-pyridinium-bis-trifluormethansulfonimid
1-Butyl-3-methylimidazolium nonafluoropentanoat
1-Octyl-3-methylimidazolium nonafluoropentanoat
1-Butyl-3-methylimidazolium trifluormethansulfonat
1-Butyl-3-methylimidazolium trifluormethansulfonat

Bei dem erfindungsgemäßen Verfahren können zusätzliche organische Lösungsmittel bei der Alkylierungsreaktion eingesetzt werden. Bevorzugte organische Lösungsmittel sind solche, die einen Siedepunkt unterhalb 200°C besitzen. Besonders bevorzugt sind Chlorbenzol, Methylenchlorid, Acetonitril, Toluol oder Gemische dieser organischen Lösungsmittel. Bevorzugt sind Lösungsmittel mit einem Siedepunkt von weniger als 120°C.

Das zusätzlich eingesetzte Lösungsmittel kann bei der Produktisolierung destillativ zurückgewonnen und wiederverwendet werden.

Die Erfindung wird durch die nachfolgenden Beispiele näher beschrieben.

### Beispiele

### 1. Synthese der ionische Flüssigkeit 1-Butyl-3-methy-imidazolium tetrafluoroborat nach Stand der Technik (Referenzbeispiel)

Nach einer Vorschrift von Wilkes (J. S. Wilkes, J. A. Levisky, R. A. Wilson, C. L. Hussey, *Inorg. Chem.,* **1982,** 21, 1263) wird in einem ersten Schritt das 1-Butyl-3-methyl-imidazoliumchloride wie folgt hergestellt. In einen 500 ml Schlenkkolben mit Rückflusskühler werden 46,0 g (0,56 mol) 1-Methylimidazol und 77,8 g (0,84 mol) Butylchlorid zusammengegeben und bei 80°C ca. 3 Tage gerührt. Anschließend wir das überschüssige Butylchlorid abdekantiert und das Produkt bei 80°C am HV getrocknet. Man erhält 80,2 g des weiß, gelblichen Feststoff, 1-Butyl-3-methyl-imidazoliumchloride.

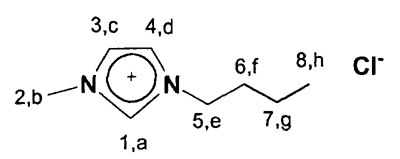

^{**1**}**H-NMR (300 MHz, CDCl**_{**3**}**):** δ=9.9 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,2H,H_{g}); δ=0.8 ppm (t,3H,Hₕ).

^{**13**}**C-NMR (75 MHz, CDCl**_{**3**}**):** δ=137 ppm (C1); δ=124-122 ppm (C3,C4); δ=50 ppm (C5); δ=36 ppm (C2); δ=33 ppm (C6); δ=20 ppm (C7); δ=14 ppm (C8).

In einem zweiten Schritt wird nach einer Vorschrift Wilkes (P.A.Z. Suarez, J.E.L. Dullius, S. Einloft, R.F. de Souza, J. Dupont, *Polyhedron,* **1996,** *15,* 1217-1219) aus dem 1-Butyl-3-methyl-imidazoliumchlorid durch Anionenaustausch das 1-Butyl-3-methyl-imidazolium tetrafluoroborat hergestellt.

Die im ersten Schritt hergestellten 80,2 g (0,46 mol) 1-Butyl-3-methylimidazoliumchloride werden in ca. 200 ml Aceton gelöst und mit 75,62 g (0,69 mol) Natriumtetrafluoroborat versetzt und für ca. 1 Woche bei 70°C gerührt. Der entstandene Feststoff wird über eine Schutzgasfritte abfiltriert und das Filtrat am Rotationsverdampfer vom Lösungsmittel befreit und über Nacht bei 60°C am HV getrocknet. Man erhält 1-Butyl-3-methyl-imidazoliumtetrafluoroborat in 74 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Ein weißer Niederschlag spricht für eine nicht vollständige Chloridfreiheit des Produktes.

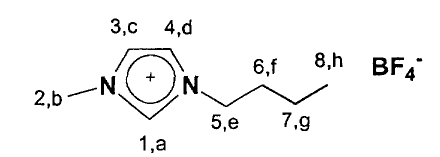

^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).

^{**13**}**C-NMR (75 MHz, CDCl**_{**3**}**):** 14,1 (a); 20,3-36,8 (b, c, d); 50,4 (h); 123,2-125,0 (f,g); 137,8 (e).

^{**19**}**F-NMR (281 MHz, CDCl**_{**3**}**):** 148,5-150,8 (m)

### 2. Synthese der ionische Flüssigkeit 1-Butyl-3-methy-imidazolium tetrafluoroborat nach dem neuen Verfahren gemäß dieser Erfindung

In einen 2 I Schlenkkolben werden 620,5 g (5 mol) 1-Butylimidazol vorgelegt und 630,6 g (5 mol) Dimethylsulfat portionsweise zugegeben. Der Ansatz wird danach 15 Minuten nachgerührt. Der Kolbeninhalt wird in ein 5l Becherglas überführt und zu einer Mischung von 548,0 g (5 mol) Natriumtetrafluoroborat in 2 Liter Wasser gegeben. Die wäßrige Phase wird nun viermal mit je 1 Liter CH₂Cl₂ extrahiert, die organischen Phasen vereinigt und von Methylenchlorid befreit. Das Produkt wird über Nacht bei 60°C am HV getrocknet. Man erhält 1-Butyl-3-methylimidazolium tetrafluoroborat in 84 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Chloridresten.

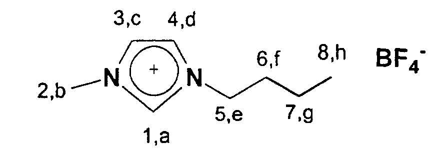

^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).

^{**13**}**C-NMR (75 MHz, CDCl**_{**3**}**):** 14,1 (a); 20,3-36,8 (b, c, d); 50,4 (h); 123,2-125,0 (f,g); 137,8 (e).

^{**19**}**F-NMR (281 MHz, CDCl**_{**3**}**):** 148,5-150,8 (m)

### 3. Synthese der ionische Flüssigkeit 1,3 Dimethyl-imidazolium methylsulfat

In einen 1 l Schlenkkolben werden 246,3 g (3mol) 1-Methylimidazol vorgelegt und 378,3 g (3 mol) Dimethylsulfat portionsweise zugegeben. Danach wird 15 min nachgerührt.

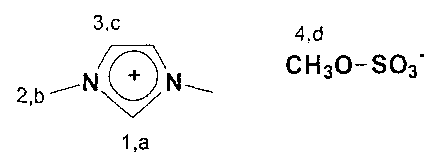

^{**1**}**H-NMR (300 MHz, CDCl**_{**3**}**):** δ=9.2 ppm (s,1H,Hₐ); δ=6.9 ppm (d,2H,H_{c}); δ=3.3 ppm (s,6H,H_{b}); δ=2.9 ppm (s,3H,H_{d}).

^{**13**}**C-NMR (75 MHz, CDCl**_{**3**}**):** δ=136 ppm (C1); δ=123 ppm (C3); δ=53 ppm (C4); δ=35 ppm (C2).

### 4. Synthese der ionische Flüssigkeit 1-Butyl-3-methylimidazoliummethylsulfat (Verfahren 1)

In einen 1 I Schlenkkolben werden 372.3g (3mol) 1-Butylimidazol vorgelegt und 378,3 g (3 mol) Dimethylsulfat portionsweise zugegeben. Danach wird 15 min nachgerührt.

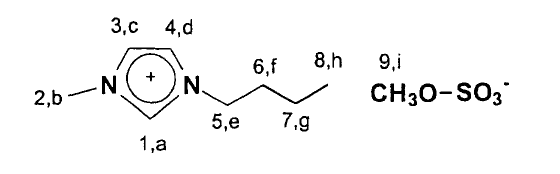

^{**1**}**H-NMR (300 MHz, CDCl**_{**3**}**):** δ=9.9 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=2.9 ppm (s,3H,Hᵢ); δ=1.6 ppm (m,2H,H_{f}); δ=1.2 ppm (m,2H,H_{g}); δ=0.8 ppm (t,3H,Hₕ).

^{**13**}**C-NMR (75 MHz, CDCl**_{**3**}**):** δ=137 ppm (C1); δ=124-122 ppm (C3,C4); δ=53 ppm (C9); δ=50 ppm; (C5); δ=36 ppm (C2); δ=33 ppm (C6); δ=20 ppm (C7); δ=14 ppm (C8).

### Synthese der ionische Flüssigkeit 1-Butyl-3-methylimidazolium methylsulfat (Verfahren 2)

In einen 1 I Schlenkkolben werden 246,3 g (3mol) 1-Methylimidazol vorgelegt und 630,6 g (3 mol) Dibutylsulfat portionsweise zugegeben. Danach wird 15 min nachgerührt.

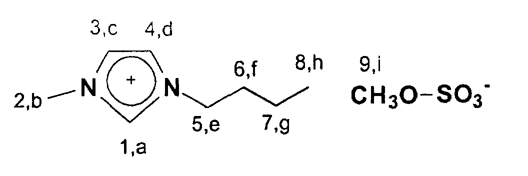

^{**1**}**H-NMR (300 MHz, CDCl**_{**3**}**):** δ=9.9 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=2.9 ppm (s,3H,H;); δ=1.6 ppm (m,2H,H_{f}); δ=1.2 ppm (m,2H,H_{g}); δ=0.8 ppm (t,3H,Hₕ).

^{**13**}**C-NMR (75 MHz, CDCl**_{**3**}**):** δ=137 ppm (C1); δ=124-122 ppm (C3,C4); δ=53 ppm (C9); δ=50 ppm; (C5); δ=36 ppm (C2); δ=33 ppm (C6); δ=20 ppm (C7); δ=14 ppm (C8).

### 5. Synthese der ionische Flüssigkeit 1-Ethyl-3-methy-imidazolium hexafluorophosphat

In einen 2 l Schlenkkolben werden 410,5 g (5 mol) 1-Methylimidazol vorgelegt und 770,95g (5 mol) Diethylsulfat portionsweise zugegeben. Der Ansatz wird danach 15 Minuten nachgerührt. Der Kolbeninhalt wird danach in ein 5l Becherglas überführt und zu einer Mischung von 839,7g (5 mol) Natriumhexafluorophosphat in 2 Liter Wasser gegeben. Sofort bildet sich ein weißer Feststoff, der durch Filtration isoliert wird. Die wäßrige Phase wird noch zweimal mit je 1 Liter CH₂Cl₂ extrahiert, die organischen Phasen vereinigt und von Methylenchlorid befreit. Das Produkt wird über Nacht bei 60°C am HV getrocknet. Man erhält 1-Ethyl-3-methyl-imidazolium hexafluorophosphat in 82 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

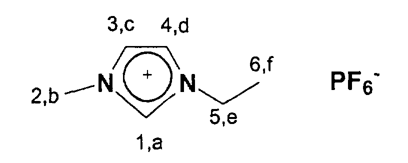

^{**1**}**H-NMR (300 MHz, CDCl**_{**3**}**):** δ=9.6 ppm (s,1H,Hₐ); δ=7.26 ppm (d,1H,H_{c}); δ=7.24 ppm (d,1H,H_{d}); δ=3.9 ppm (q,2H,Hₑ); δ=3.6 ppm (s,3H,H_{b}); δ=1.2 ppm (t,3H,H_{f}).

^{**13**}**C-NMR (75 MHz, CDCl**_{**3**}**):** δ=137 ppm (C1); δ=124-122 ppm (C3,C4); δ=45 ppm (C5); δ=36 ppm (C2); δ=16 ppm (C6).

^{**31**}**P-NMR (121 Mhz):** -143.08 (Heptett, J=710 Hz).

^{**19**}**F-NMR (281 Mhz):** - 72.5 (d, J=710 Hz).

### 6. Synthese der ionische Flüssigkeit 1-Butyl-3-methy-imidazolium hexafluorophosphat

In einen 2 l Schlenkkolben werden 620,5 g (5 mol) 1-Butylimidazol vorgelegt und 630,5g (5 mol) Dimethylsulfat portionsweise zugegeben. Der Ansatz wird danach 15 Minuten nachgerührt. Der Kolbeninhalt wird in ein 5l Becherglas überführt und zu einer Mischung von 839,7g (5 mol) Natriumhexafluorophosphat in 2 Liter Wasser gegeben. Sofort bildet sich das Produkt als eine zweite flüssige Phase. Nach einer Phasentrennung wird die wässrige Phase noch einmal mit 1 Liter CH₂Cl₂ extrahiert, und das organische Extrakt von Methylenchlorid befreit. Beide Produktfraktionen werden vereinigt und über Nacht bei 60°C am HV getrocknet. Man erhält 1-Butyl-3-methylimidazoliumhexafluorophosphat in 92 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Chloridresten.

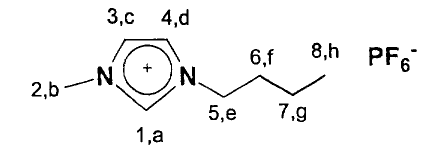

^{**1**}**H-NMR (300 MHz, CDCl**_{**3**}**):** 0,77 (3 H, tr, J=5,8 Hz, Hₐ); 1,05-1,17 (2 H, m, H_{b}); 1,50-1,59 (2 H, m, H_{c}); 3,51 (3 H, s, Hₕ); 3,75 (2 H, tr, J=5,8 Hz, H_{d}); 6,95-7,26 (je 1 H, s, H_{f,g}); 7,97 (1 H, s, Hₑ).

^{**13**}**C-NMR (75 MHz, CDCl**_{**3**}**):** 13,3 (a); 19,4-35,7 (b, c, d); 49,5 (h); 122,2-123,5 (f,g); 135,7 (e).

^{**31**}**P-NMR (121 MHz, CDCl**_{**3**}**):** -143,08 (Heptett, J=710Hz)

^{**19**}**F-NMR (281 MHz, CDCl**_{**3**}**):** -72,5 (d, J=710 Hz)

### 7. Synthese der ionische Flüssigkeit 1-Octyl-3-methy-imidazolium tetrafluoroborat

In einen 2 I Schlenkkolben werden 830,5 g (5 mol) 1-Octylimidazol vorgelegt und 630,5g (5 mol) Dimethylsulfat portionsweise zugegeben. Der Ansatz wird danach 15 Minuten nachgerührt. Der Kolbeninhalt wird danach in ein 5l Becherglas überführt und zu einer Mischung von 548,0g (5 mol) Natriumtetrafluoroborat in 2 Liter Wasser gegeben. Sofort bildet sich das Produkt als zweite, flüssige Phase.. Nach einer Phasentrennung wird die wässrige Phase noch einmal mit 1 Liter CH₂Cl₂ extrahiert, und das organische Extrakt von Methylenchlorid befreit. Beide Produktfraktionen werden vereinigt und über Nacht bei 60°C am HV getrocknet. Man erhält 1-Octyl-3-methylimidazoliumtetrafluoroborat in 94 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Chloridresten.

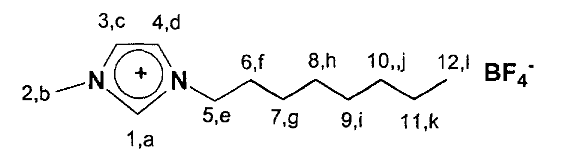

^{**1**}**H-NMR (300 MHz, CDCl**_{**3**}**):** δ=9.3 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,10H,H_{g,h,i,j,k}); δ=0.8 ppm (t,3H,Hₗ).

^{**13**}**C-NMR (75 MHz, CDCl**_{**3**}**):** δ=137 ppm (C1); δ=124-122 ppm (C3,C4); δ=48 ppm (C5); δ=35 ppm (C2); δ=29-24 ppm (C6-C10); δ=20 ppm (C11); δ=13 ppm (C12).

^{**19**}**F-NMR (281 MHz, CDCl**_{**3**}**):** δ= -151,3 ppm (d, ¹J(BF)= 22 Hz)

### 8. Synthese der ionische Flüssigkeit 4-Methyl-N-butyl-pyridinium hexafluorophosphat

In einen 2 l Schlenkkolben werden 465,7 g (5 mol) 4-Picolin vorgelegt und 1051,0 (5 mol) Dibutylsulfat portionsweise zugegeben. Der Ansatz wird danach 15 Minuten nachgerührt. Der Kolbeninhalt wird danach in ein 5l Becherglas überführt und zu einer Mischung von 839,7g (5 mol) Natriumhexafluorophosphat in 2 Liter Wasser gegeben. Sofort bildet sich ein weißer Feststoff, der durch Filtration isoliert wird. Die wässrige Phase wird noch zweimal mit je 1 Liter CH₂Cl₂ extrahiert, die organischen Phasen vereinigt und von Methylenchlorid befreit. Das Produkt wird über Nacht bei 60°C am HV getrocknet. Man erhält 1-Ethyl-3-methyl-imidazoliumhexafluorophosphat in 82 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

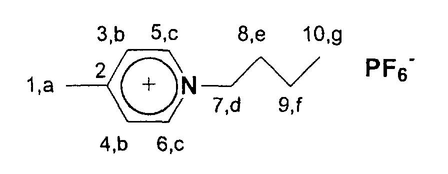

^{**1**}**H-NMR (300 MHz, CDCl**_{**3**}**:** 0,79 (3 H, tr, J=7,45 Hz, Hₐ); 1,24 (2 H, mult, H_{b}); 1,81 (2 H, mult, H_{c}); 2,50 (3 H, s, Hₖ); 4,43 (2 H, tr, J=7,2 Hz, H_{d};); 7,72 ( 2 H, H_{g,h}); 8,56 (2 H, H_{e,f}).

^{**13**}**C-NMR (75 Mhz, CDCl**_{**3**}**:** 13,1 (a); 18,9 (b); 21,6 (k); 33,0 (c); 60,8 (d); 128,8 (g,h); 143,3 (i); 159,3 (e,f).

^{**31**}**P-NMR (121 MHz, CDCl**_{**3**}**):** -143,08 (Heptett, J=710Hz)

^{**19**}**F-NMR (281 MHz, CDCl**_{**3**}**):** -72,5 (d, J=710 Hz)

### 9. Synthese der ionische Flüssigkeit 1-Butyl-3-methyl-imidazoliumnonafluorobutansulfonat

In einen 5l Reaktionsgefäß werden 620,5 g (5 mol) 1-Butylimidazol vorgelegt und 630,5g (5 mol) Dimethylsulfat portionsweise zugegeben. Der Ansatz wird danach 15 Minuten nachgerührt. Der Reaktorinhalt wird mit einer Mischung von 1690,0g (5 mol) Kaliumnonafluorobutansulfonat in 2 Liter Wasser versetzt. Sofort bildet sich das Produkt als eine zweite flüssige Phase. Nach einer Phasentrennung wird die wässrige Phase noch einmal mit 1 Liter CH₂Cl₂ extrahiert, und das organische Extrakt von Methylenchlorid befreit. Beide Produktfraktionen werden vereinigt und über Nacht bei 60°C am HV getrocknet. Man erhält 1-Butyl-3-methylimidazolium-nonafluorobutansulfonat in 90 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

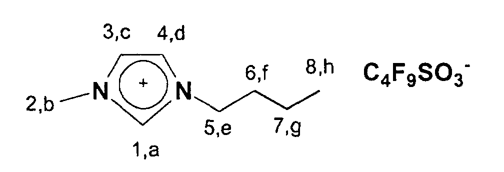

^{**1**}**H-NMR (300 MHz, Aceton-d**_{**3**}**):** 0,87 (3 H, tr, J=9,2 Hz, Hₕ); 1,29-1,38 (2 H, m, H_{g}); 1,90-2,00 (2 H, m, H_{f}); 4,04 (3 H, s, H_{b}); 4,35 (2 H, tr, J=9,3 Hz, Hₑ); 7,71;7,78 (je 1 H, s, H_{c,d}); 9,11 (1 H, s, Hₐ) ppm.

### 10. Synthese der ionische Flüssigkeit 1-Butyl-3-methylimidazoliumtrifluormethylsulfonat

In einen 2 I Schlenkkolben werden 620,5 g (5 mol) 1-Butylimidazol vorgelegt und 630,5g (5 mol) Dimethylsulfat portionsweise zugegeben. Der Ansatz wird danach 15 Minuten nachgerührt. Der Kolbeninhalt wird in ein 5l Becherglas überführt und zu einer Mischung von 860,3g (5 mol) Natriumtrifluormethansulfonat in 2 Liter Wasser gegeben. Sofort bildet sich das Produkt als eine zweite flüssige Phase. Nach einer Phasentrennung wird die wässrige Phase noch einmal mit 1 Liter CH₂Cl₂ extrahiert, und das organische Extrakt von Methylenchlorid befreit. Beide Produktfraktionen werden vereinigt und über Nacht bei 60°C am HV getrocknet. Man erhält 1-Butyl-3-methylimidazolium-trifluormethylsulfonat in 84 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

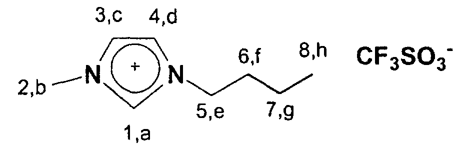

^{**1**}**H-NMR (300 MHz, Aceton-d**_{**3**}**):** 0,87 (3 H, tr, J=9,2 Hz, Hₕ); 1,29-1,38 (2 H, m, H_{g}); 1,90-2,00 (2 H, m, H_{f}); 4,04 (3 H, s, H_{b}); 4,35 (2 H, tr, J=9,3 Hz, Hₑ); 7,71;7,78 (je 1 H, s, H_{c,d}); 9,11 (1 H, s, Hₐ) ppm.

### 11. Synthese der ionische Flüssigkeit 1-Butyl-3-methylimidazolium-bis-(trifluormethylsulfon)imid

In einen 5 I Reaktionsgefäß werden 620,5 g (5 mol) 1-Butylimidazol vorgelegt und 630,5g (5 mol) Dimethylsulfat portionsweise zugegeben. Der Ansatz wird danach 15 Minuten nachgerührt. Dazu gibt man eine Mischung von 1435,3g (5 mol) Lithium-bis-trifluormethansulfonimid in 2 Liter Wasser. Sofort bildet sich das Produkt als eine zweite flüssige Phase. Nach einer Phasentrennung wird das Produkt bei 60°C am HV getrocknet. Man erhält 1-Butyl-3-methylimidazoliumbis(trifluormethylsulfon)imid in 98 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

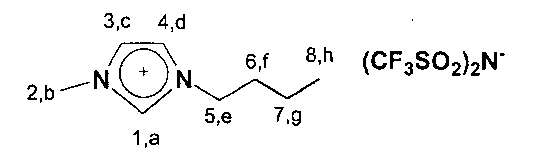

^{**1**}**H-NMR (300 MHz, Aceton-d**_{**3**}**):** 0,95 (3 H, tr, J=9,2 Hz, Hₕ); 1,33-1,39 (2 H, m, H_{g}); 1,90-2,00 (2 H, m, H_{f}); 4,07 (3 H, s, H_{b}); 4,37 (2 H, tr, J=9,3 Hz, Hₑ); 7,71;7,76 (je 1 H, s, H_{c,d}); 9,02 (1 H, s, Hₐ) ppm.

### 12. Synthese der ionische Flüssigkeit 1-Butyl-3-methylimidazoliumtrifluoracetat

In einen 5 I Reaktionsgefäß werden 620,5 g (5 mol) 1-Butylimidazol vorgelegt und 630,5g (5 mol) Dimethylsulfat portionsweise zugegeben. Der Ansatz wird danach 15 Minuten nachgerührt. Dazu gibt man eine Mischung von 680,0g (5 mol) Natriumtrifluoracetat in 2 Liter Wasser.

Sofort bildet sich das Produkt als eine zweite flüssige Phase. Nach der Phasentrennung wird die wässrige Phase 3 mal mit je einem Liter CH₂Cl₂ extrahiert. Die vereinigten organischen Phasen werden am Rotationsverdampfer von CH₂Cl₂ befreit und der Rückstand wird mit der abgetrennten ionischen Flüssigkeit aus der Phasentrennung vereint. Das Produkt wird bei 60°C am HV getrocknet. Man erhält 1-Butyl-3-methylimidazolium-trifluoracetat in 75 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Silberchlorid-Niederschlags spricht für die komplette Abwesenheit von Halogenidionen.

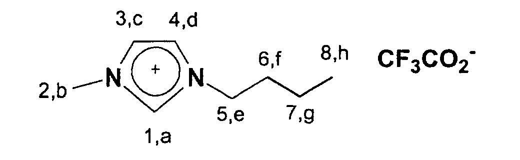

^{**1**}**H-NMR (300 MHz, Aceton-d**_{**3**}**):** 0,94 (3 H, tr, J=9,3 Hz, Hₕ); 1,37 (2 H, m, H_{g}); 1,92 (2 H, m, H_{f}); 4,07 (3 H, s, H_{b}); 4,37 (2 H, tr, J=9,3 Hz, Hₑ); 7,78;7,84 (je 1 H, s, H_{c,d}); 9,88 (1 H, s, Hₐ) ppm.

## Patentansprüche

1. Verfahren zur Herstellung ionischer Flüssigkeiten der allgemeinen Formel
[A]ₙ⁺[Y]ⁿ⁻,
wobei n = 1 oder 2 ist und
das Anion [Y]ⁿ⁻ ausgewählt ist aus der Gruppe bestehend aus Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprat, Sulfat ([SO₄]²⁻), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann,
das Kation [A]⁺ ist ausgewählt aus
- quarternären Ammonium-Kationen der allgemeinen Formel
[NR¹R²R³R]⁺,
- Phosphonium-Kationen der allgemeinen Formel
[PR¹R²R³R]⁺,
- Imidazolium-Kationen der allgemeinen Formel 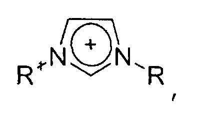 wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinium-Kationen der allgemeinen Formel 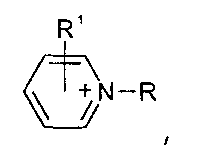 wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolium-Kationen der allgemeinen Formel 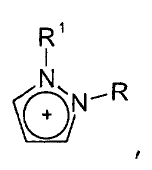 wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- und Triazolium-Kationen der allgemeinen Formel 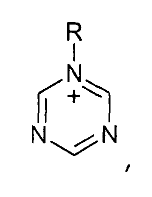 wobei der Triazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
**dadurch gekennzeichnet, dass** die zugrundeliegenden Amine, Phosphine, Imidazole, Pyridine, Triazole und Pyrazole mit einem Disulfat der allgemeinen Formel R⁴-SO₄-R⁵ alkyliert werden, wobei die Reste R⁴ und R⁵ unabhängig voneinander ausgewählt sein können aus
- linearen oder verzweigten, gesättigten oder ungesättigten, aliphatischen oder alicyclischen Alkylgruppen mit 1 bis 24 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 24 Kohlenstoffatomen im Arylrest, die gegebenenfalls mit wenigstens einer C₁-C₆-Alkylgruppe und/oder einem Halogenenatomen substituiert sein können, alkyliert werden und anschließend ein Austausch des Sulfatanions R⁴-SO₄⁻ oder R⁵-SO₄⁻ durch das oben definierte Anion [Y]⁻ oder [Y]²⁻ erfolgt.

2. Verfahren nach Anspruch 1 **dadurch gekennzeichnet, dass** der Anionenaustausch mit Metallsalzen der Formel M^{m+}[Y]⁻ₘ, M₂⁺[Y]²⁻ oder M²⁺[Y]²⁻ erfolgt, wobei die Anionen [Y]⁻ und [Y]²⁻ ausgewählt sind aus der in Anspruch 1 definierten Gruppe und m = 1, 2 oder 3 ist.

3. Verfahren nach Anspruch 2 **dadurch gekennzeichnet, dass** das Metall-Ion M ausgewählt ist aus der Gruppe der Alkali- oder Erdalkalimetalle, Blei oder Silber.

4. Verfahren nach irgendeinem der Ansprüche 1 bis 3, **dadurch gekennzeichnet, dass** die Zugabe des zum Anionenaustausch verwendeten Metallsalzes der Formel M^{m+}[Y]⁻ₘ , M₂⁺[Y]²⁻ oder M²⁺[Y]²⁻ zur Reaktionsmischung nach der Alkylierung mit dem Disulfat der allgemeinen Formel R⁴-SO₄-R⁵ erfolgt.

5. Verfahren nach Anspruch 1, **dadurch gekennzeichnet, dass** ein der Anionenaustausch mit dem Metallsalz der Formel M^{m+}[Y]⁻ₘ , M₂+[Y]²⁻ oder M²⁺[Y]²⁻ in wässriger Lösung erfolgt.

6. Verfahren nach Anspruch 5, **dadurch gekennzeichnet, dass** das molare Verhältnis des Disulfats der allgemeinen Formel R⁴-SO₄-R⁵ zu den zugrundeliegenden Aminen, Phosphinen, Imidazolen-, Triazolen-, Pyrazolen-, oder Pyridinen zwischen 1 und 5 liegt.

7. Verfahren nach irgendeinem der Ansprüche 1 bis 6, **dadurch gekennzeichnet, dass** die Reaktion in Substanz oder unter Zusatz eines organischen Lösungsmittels durchgeführt wird.

8. Verfahren nach Anspruch 6, **dadurch gekennzeichnet, dass** als Lösungsmittel Chlorbenzol, Methylenchlorid, Acetonitril, Toluol, Tetrahydrofuran, Diethylether, oder Gemische dieser organischen Lösungsmittel eingesetzt werden.

9. Verfahren nach Anspruch 1 und 8 **dadurch gekennzeichnet, dass** das molare Verhältnis des zugesetzten organischen Lösungsmittels zu den zugrundeliegenden Aminen, Phosphinen, Imidazolen, Triazolen, Pyrazolen oder Pyridinen zwischen 0,5 und 20 beträgt.

10. Verfahren nach irgendeinem der Ansprüche 1 bis 9, **dadurch gekennzeichnet, dass** die Reaktion dem Disulfat der allgemeinen Formel R⁴-SO₄-R⁵ bei einer Temperatur zwischen -10 und 250°C durchgeführt wird.

11. Verfahren nach irgendeinem der Ansprüche 1 bis 10, **dadurch gekennzeichnet, dass** die Reaktionszeit 1 min bis 8h beträgt.

12. Verfahren nach irgendeinem der Ansprüche 1 bis 11, **dadurch gekennzeichnet, dass** der Anionenaustausch bei einer Temperatur zwischen -10 und 100°C durchgeführt wird.

13. Verfahren nach irgendeinem der Ansprüche 1 bis 12, **dadurch gekennzeichnet, dass** die zum Anionenaustausch eingesetzte wässrige Lösung das Metallsalz in einer Konzentration von 0,1 bis 99 Gew.-% enthält.

14. Verfahren nach irgendeinem der Ansprüche 1 bis 13, **dadurch gekennzeichnet, dass** das durch die Alkylierung erhaltene Sulfatsalz [A]⁺[R⁴-SO₄]⁻ oder [A]⁺[R⁵-SO₄]⁻ vor dem Anionenaustausch isoliert wird oder ohne vorherige Isolierung mit der Lösung des Metallsalzes der allgemeinen Formel M^{m+}[Y]⁻ₘ, M₂⁺[Y]²⁻ oder M²⁺[Y]²⁻ versetzt wird.

15. Verfahren nach irgendeinem der Ansprüche 1 bis 14, **dadurch gekennzeichnet, dass** R⁴ = R⁵ ist.

## Claims

1. A process for the preparation of ionic liquids of general formula
[A]ₙ⁺[Y]ⁿ⁻,
wherein n = 1 or 2; and
the anion [Y]ⁿ⁻ is selected from the group consisting of tetrafluoroborate ([BF₄]⁻), tetrachloroborate ([BCl₄]⁻), hexafluorophosphate ([PF₆]⁻), hexafluoroantimonate ([SbF₆]⁻), hexafluoroarsenate ([AsF₆]⁻), tetrachloroaluminate ([AlC₄]⁻), trichlorozincate ([ZnCl₃]⁻), dichlorocuprate, sulfate ([SO₄]²⁻), carbonate ([CO₃]²⁻), fluorosulfonate, [R'-COO]⁻, [R'-SO₃]⁻ or [(R'-SO₂)₂N]⁻, and R' is a linear or branched aliphatic or alicyclic alkyl containing from 1 to 12 carbon atoms, or a C₅-C₁₈ aryl, C₅-C₁₈-aryl-C₁-C₆-alkyl or C₁-C₆-alkyl-C₅-C₁₈-aryl residue which may be substituted with halogen atoms;
the cation [A]⁺ is selected from
- quaternary ammonium cations of general formula
[NR¹R²R³R]⁺,
- phosphonium cations of general formula
[PR¹R²R³R]⁺,
- imidazolium cations of general formula 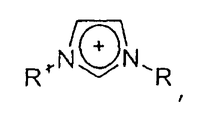 wherein the imidazole nucleus may be substituted with at least one group selected from C₁-C₆ alkyl, C₁-C₆ alkoxy, C₁-C₆ aminoalkyl, C₅-C₁₂ aryl or C₅-C₁₂-aryl-C₁-C₆-alkyl groups;
- pyridinium cations of general formula 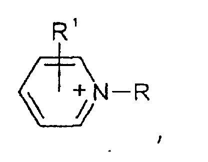 wherein the pyridine nucleus may be substituted with at least one group selected from C₁-C₆ alkyl, C₁-C₆ alkoxy, C₁-C₆ aminoalkyl, C₅-C₁₂ aryl or C₅-C₁₂-aryl-C₁-C₆-alkyl groups;
- pyrazolium cations of general formula 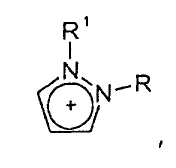 wherein the pyrazole nucleus may be substituted with at least one group selected from C₁-C₆ alkyl, C₁-C₆ alkoxy, C₁-C₆ aminoalkyl, C₅-C₁₂ aryl or C₅-C₁₂-aryl-C₁-C₆-alkyl groups; and
- triazolium cations of general formula 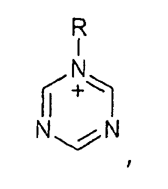 wherein the triazole nucleus may be substituted with at least one group selected from C₁-C₆ alkyl, C₁-C₆ alkoxy, C₁-C₆ aminoalkyl, C₅-C₁₂ aryl or C₅-C₁₂-aryl-C₁-C₆-alkyl groups; and
the residues R¹, R², R³ are independently selected from the group consisting of
- hydrogen;
- linear or branched, saturated or unsaturated, aliphatic or alicyclic alkyl groups having from 1 to 20 carbon atoms;
- heteroaryl, heteroaryl-C₁-C₆-alkyl groups having from 3 to 8 carbon atoms in the heteroaryl residue and at least one heteroatom selected from N, O and S which may be substituted with at least one group selected from C₁-C₆ alkyl groups and/or halogen atoms;
- aryl, aryl-C₁-C₆-alkyl groups having from 5 to 12 carbon atoms in the aryl residue which may be optionally substituted with at least one C₁-C₆ alkyl group and/or halogen atom;
**characterized in that** the underlying amines, phosphines, imidazoles, pyridines, triazoles and pyrazoles are alkylated with a disulfate of general formula R⁴-SO₄-R⁵, wherein the residues R⁴ and R⁵ may be independently selected from
- linear or branched, saturated or unsaturated, aliphatic or alicyclic alkyl groups having from 1 to 24 carbon atoms;
- heteroaryl-C₁-C₆-alkyl groups having from 3 to 8 carbon atoms in the aryl residue and at least one heteroatom selected from N, O and S which may be substituted with at least one C₁-C₆ alkyl group and/or halogen atom;
- aryl-C₁-C₆-alkyl groups having from 5 to 24 carbon atoms in the aryl residue which may be optionally substituted with at least one C₁-C₆ alkyl group and/or halogen atom;
followed by exchanging the sulfate anion R⁴-SO₄⁻ or R⁵-SO₄⁻ by the anion [Y]⁻ or [Y]²⁻ as defined above.

2. The process according to claim 1, **characterized in that** the anion exchange is effected with metal salts of formula M^{m+}[Y]⁻ₘ, M2⁺[Y]²⁻ or M²⁺[Y]²⁻, wherein the anions [Y]⁻ or [Y]²⁻ are selected from the group as defined in claim 1, and m = 1, 2 or 3.

3. The process according to claim 2, **characterized in that** the metal ion M is selected from the group of alkali or alkaline earth metals, lead or silver.

4. The process according to any of claims 1 to 3, **characterized in that** the addition of the metal salt of formula M^{m+}[Y]⁻ₘ, M₂⁺[Y]²⁻ or M²⁺[Y]²⁻ used for anion exchange to the reaction mixture is effected after the alkylation with the disulfate of general formula R⁴-SO₄-R⁵.

5. The process according to claim 1, **characterized in that** the anion exchange with the metal salt of formula M^{m+}[Y]⁻ₘ, M₂⁺[Y]²⁻ or M²⁺[Y]²⁻ is effected in aqueous solution.

6. The process according to claim 5, **characterized in that** the molar ratio of the disulfate of general formula R⁴-SO₄-R⁵ to the underlying amines, phosphines, imidazoles, triazoles, pyrazoles or pyridines is between 1 and 5.

7. The process according to any of claims 1 to 6, **characterized in that** the reaction is performed neat or with the addition of an organic solvent.

8. The process according to claim 6, **characterized in that** chlorobenzene, methylene chloride, acetonitrile, toluene, tetrahydrofuran, diethyl ether or mixtures of such organic solvents are employed as the solvent.

9. The process according to claims 1 and 8, **characterized in that** the molar ratio of the added organic solvent to the underlying amines, phosphines, imidazoles, triazoles, pyrazoles or pyridines is between 0.5 and 20.

10. The process according to any of claims 1 to 9, **characterized in that** the reaction with the disulfate of general formula R⁴-SO₄-R⁵ is performed at a temperature of between -10 and 250°C.

11. The process according to any of claims 1 to 10, **characterized in that** the reaction time is from 1 min to 8 h.

12. The process according to any of claims 1 to 11, **characterized in that** the anion exchange is performed at a temperature of between -10 and 100°C.

13. The process according to any of claims 1 to 12, **characterized in that** the aqueous solution employed for anion exchange contains the metal salt at a concentration of from 0.1 to 99% by weight.

14. The process according to any of claims 1 to 13, **characterized in that** the sulfate salt [A]⁺[R⁴-SO₄]⁻ or [A]⁺[R⁵-SO₄]⁻ obtained by alkylation is isolated before the anion exchange, or is admixed with the solution of the metal salt of general formula M^{m+}[Y]⁻ₘ, M₂⁺[Y]²⁻ or M²⁺[Y]²⁻ without prior isolation.

15. The process according to any of claims 1 to 14, **characterized in that** R⁴ = R⁵.

## Revendications

1. Procédé de fabrication de fluides ioniques de formule générale
[A]ₙ⁺[Y]ⁿ⁻,
sachant que n = 1 ou 2 et
l'anion [Y]ⁿ⁻ est choisi dans le groupe composé du tétrafluoroborate ([BF₄]⁻), du tétrachloroborate ([BCl₄]⁻), de l'hexafluorophosphate ([PF₆]⁻), de l'hexafluoroantimonate ([SbF₆]⁻), de l'hexafluoroarsenate ([AsF₆]⁻), du tétrachloroaluminate ([AlCl₄]⁻), du trichlorozincate ([ZnCl₃]⁻), du dichlorocuprate, du sulfate ([SO₄]²⁻), du carbonate ([CO₃])²⁻, du fluorosulfonate, de [R'-COO]⁻, [R'-SO₃]⁻ ou [(R'-SO₂)₂N]⁻, et R' est un radical alkyle aliphatique ou alicyclique linéaire ou ramifié contenant de 1 à 12 atomes de carbone, ou un radical aryle en C₅-C₁₈ ou un radical aryle C₅-C₁₈-alkyle C₁-C₆ ou un radical alkyle C₁-C₆-aryle C₅-C₁₈, pouvant être substitué par un atome d'halogène,
le cation [A]⁺ est choisi parmi
- des cations ammonium quaternaire de formule générale
[NR¹R²R³R]⁺,
- des cations phosphonium de formule générale
[PR¹R²R³R]⁺,
- des cations imidazolium de formule générale 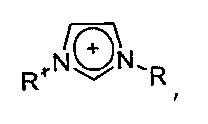 le noyau imidazole pouvant être substitué par au moins un groupe choisi parmi des groupes alkyle en C₁-C₆, alcoxy en C₁-C₆, aminoalkyle en C₁-C₆, aryle en C₅-C₁₂ ou aryle C₅-C₁₂-alkyle C₁-C₆,
- des cations pyridinium de formule générale 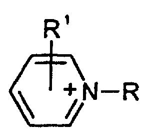 le noyau pyridine pouvant être substitué par au moins un groupe choisi parmi des groupes alkyle en C₁-C₆, alcoxy en C₁-C₆, aminoalkyle en C₁-C₆, aryle en C₅-C₁₂ ou aryle C₅-C₁₂-alkyle C₁-C₆,
- des cations pyrazolium de formule générale 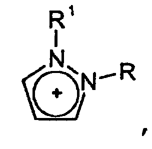 le noyau pyrazole pouvant être substitué par au moins un groupe choisi parmi des groupes alkyle en C₁-C₆, alcoxy en C₁-C₆, aminoalkyle en C₁-C₆, aryle en C₅-C₁₂ ou aryle C₅-C₁₂-alkyle C₁-C₆,
- et des cations triazolium de formule générale 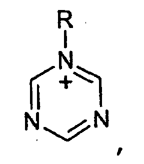 le noyau triazole pouvant être substitué par au moins un groupe choisi parmi des groupes alkyle en C₁-C₆, alcoxy en C₁-C₆, aminoalkyle en C₁-C₆, aryle en C₅-C₁₂ ou aryle C₅-C₁₂-alkyle C₁-C₆,
et les radicaux R¹, R², R³ étant choisis indépendamment les uns des autres dans le groupe consistant en
- l'hydrogène
- des groupes alkyles aliphatiques ou alicycliques linéaires ou ramifiés, saturés ou insaturés avec de 1 à 20 atomes de carbone ;
- des groupes hétéroaryles ou hétéroaryles C₁-C₆-alkyles avec de 3 à 8 atomes de carbone dans le radical hétéroaryle et au moins un hétéroatome choisi parmi N, O et S, pouvant être substitué par au moins un groupe choisi parmi des groupes alkyles en C₁-C₆ et/ou des atomes d'halogène ;
- des groupements aryles, aryle-alkyle en C₁-C₆ avec de 5 à 12 atomes de carbone dans le radical aryle, pouvant être le cas échéant substitués par au moins un groupe alkyle en C₁-C₆ et/ou un atome d'halogène ;
**caractérisé en ce que** l'amine, la phosphine, l'imidazole, la pyridine, le triazole et le pyrazole servant de base sont alkylés avec un disulfate de formule générale R⁴-SO₄-R⁵, les radicaux R⁴ et R⁵ pouvant être choisis indépendamment l'un de l'autre parmi
- des groupes alkyles aliphatiques ou alicycliques, linéaires ou ramifiés, saturés ou insaturés, possédant de 1 à 24 atomes de carbone;
- des groupes hétéroaryle-alkyle en C₁-C₆ possédant de 3 à 8 atomes de carbone dans le radical aryle et au moins un hétéroatome choisi parmi N, O et S, pouvant être substitués par au moins groupe alkyle en C₁-C₆ et/ou des atomes d'halogène ;
- des groupes aryle-alkyle en C₁-C₆ possédant de 5 à 24 atomes de carbone dans le radical aryle, qui peuvent être éventuellement substitués par au moins un groupe alkyle en C₁-C₆ et/ou un atome d'halogène, sont alkylés, à la suite de quoi l'anion sulfate R⁴-SO₄⁻ ou R⁵-SO₄⁻ s'échange avec l'anion [Y]⁻ ou [Y]²⁻ défini ci-dessus.

2. Procédé selon la revendication 1, **caractérisé en ce que** l'échange d'anions s'effectue avec des sels métalliques de formule M^{m+}[Y]⁻ₘ, M₂⁺ [Y]²⁻ ou M²⁺[Y]²⁻, les anions [Y]⁻ et [Y]²⁻ étant choisis dans le groupe défini à la revendication 1 et m = 1, 2 ou 3.

3. Procédé selon la revendication 2, **caractérisé en ce que** l'ion métallique M est choisi dans le groupe des métaux alcalins ou alcalinoterreux, du plomb ou de l'argent.

4. Procédé selon l'une quelconque des revendications 1 à 3, **caractérisé en ce que** l'ajout au mélange réactionnel du sel métallique de formule M^{m+}[Y]⁻ₘ, M₂⁺[Y]²⁻ ou M²⁺[Y]²⁻ utilisé pour l'échange d'anions s'effectue après l'alkylation avec le disulfate de formule générale R⁴-SO₄-R⁵.

5. Procédé selon la revendication 1, **caractérisé en ce que** l'un des échanges d'anions avec le sel métallique de formule M^{m+}[Y]⁻ₘ, M₂⁺[Y]²⁻ ou M²⁺[Y]²⁻ s'effectue en solution aqueuse.

6. Procédé selon la revendication 5, **caractérisé en ce que** le rapport molaire du disulfate de formule générale R⁴-SO₄-R⁵ aux amines, phosphines, imidazoles, triazoles, pyrazoles ou pyridines de base se situe entre 1 et 5.

7. Procédé selon l'une quelconque des revendications 1 à 6, **caractérisé en ce que** la réaction est réalisée en substance ou sous ajout d'un solvant organique.

8. Procédé selon la revendication 6, **caractérisé en ce que** le solvant utilisé est le chlorobenzène, le chlorure de méthylène, l'acétonitrile, le toluène, le tétrahydrofurane, le diéthyléther ou des mélanges de ces solvants organiques.

9. Procédé selon les revendications 1 et 8, **caractérisé en ce** le rapport molaire du solvant organique ajouté se situe, par rapport aux amines, phosphines, imidazoles, triazoles, pyrazoles ou pyridines de base, entre 0,5 et 20.

10. Procédé selon l'une quelconque des revendications 1 à 9, **caractérisé en ce que** la réaction du disulfate de formule générale R⁴-SO₄-R⁵ s'effectue à une température comprise entre -10 et 250°C.

11. Procédé selon l'une quelconque des revendications 1 à 10, **caractérisé en ce que** la durée de la réaction va de 1 min à 8 h.

12. Procédé selon l'une quelconque des revendications 1 à 11, **caractérisé en ce que** l'échange d'anions s'effectue à une température comprise entre -10 et 100°C.

13. Procédé selon l'une quelconque des revendications 1 à 12, **caractérisé en ce que** la solution aqueuse utilisée pour l'échange d'anions contient le sel métallique dans une concentration allant de 0,1 à 99 % en poids.

14. Procédé selon l'une quelconque des revendications 1 à 13, **caractérisé en ce que** le sel de sulfate [A]⁺[R⁴-SO₄]⁻ ou [A]⁺[R⁵-SO₄]⁻ obtenu par l'alkylation est isolé avant l'échange d'anions ou est mélangé, sans isolation préalable, avec la solution du sel métallique de formule générale M^{m+}[Y]⁻ₘ, M₂⁺ [Y]²⁻ ou M²⁺[Y]²⁻.

15. Procédé selon l'une quelconque des revendications 1 à 14, **caractérisé en ce que** R⁴ = R⁵.
